(19) Europäisches Patentamt
European Patent Office
Office européen des brevets

(11) Publication number: **0 242 322 B1**

(12) # EUROPEAN PATENT SPECIFICATION

(45) Date of publication of patent specification: **16.12.92**

(21) Application number: **87810137.7**

(22) Date of filing: **11.03.87**

The file contains technical information submitted after the application was filed and not included in this specification

(51) Int. Cl.⁵: **C07D 231/16**, C07D 231/12, C07D 231/14, C07D 231/22, C07D 231/56, C07D 409/04, C07D 207/34, C07D 209/44, C07D 235/10, C07D 235/06, C07D 249/18

(54) **Phenylureas.**

(30) Priority: **18.03.86 US 840814**

(43) Date of publication of application: **21.10.87 Bulletin 87/43**

(45) Publication of the grant of the patent: **16.12.92 Bulletin 92/51**

(84) Designated Contracting States: **AT BE CH DE ES FR GB GR IT LI NL**

(56) References cited:
**EP-A- 0 171 544**
**GB-A- 2 134 518**

(73) Proprietor: **SANDOZ AG**
**Lichtstrasse 35**
**CH-4002 Basel(CH)**
(84) Designated Contracting States:
**BE CH ES FR GB GR IT LI NL**

(73) Proprietor: **SANDOZ-PATENT-GMBH**
**Humboldtstrasse 3**
**W-7850 Lörrach(DE)**
(84) Designated Contracting States:
**DE**

(73) Proprietor: **SANDOZ-ERFINDUNGEN Verwaltungsgesellschaft m.b.H.**
**Brunner Strasse 59**
**A-1235 Wien(AT)**
(84) Designated Contracting States:
**AT**

(72) Inventor: **Carney, Robert L.**
**2676 Emerson Street**
**Palo Alto, California 94306(US)**
Inventor: **Gruber, John M.**
**565 Willow Road 4**
**Menlo Park, California 94025(US)**
Inventor: **Lui, Alfred S.**
**716 Windsor Way**
**Redwood City, California 94062(US)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).



Rank Xerox (UK) Business Services

## Description

The present invention relates to novel substituted N-(heterocyclic-substituted phenyl)-N'-benzoylureas, to processes for producing these compounds, to intermediates therefor, to compositions thereof and to the use of the compounds for the control of pests, and in particular for the control of insects and acarids.

More particularly, the compounds of the present invention are represented by the following formula (A):

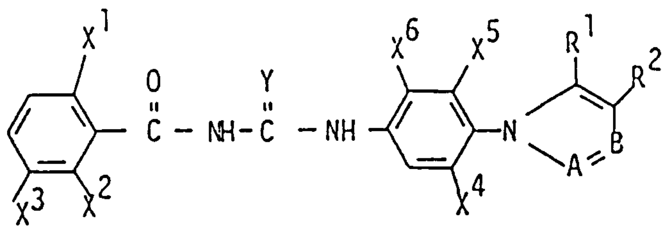

(A)

wherein, each of $X^1$, $X^2$, $X^3$ and $X^5$ is independently hydrogen, halogen or $C_{1-4}$alkyl;

$X^4$ is hydrogen, halogen, unsubstituted or halogenated $C_{1-4}$alkyl or COOR;

$X^5$ is hydrogen, halogen, $C_{1-8}$alkyl or COOR';

Y is oxygen or sulfur;

A is nitrogen or C-$R^4$;

B is nitrogen or C-$R^3$;

each of $R^1$ and $R^4$ is independently hydrogen, halogen, halogenated $C_{1-8}$-alkyl, unsubstituted or halogenated $C_{1-8}$alkoxy, unsubstitutcd or halogenated $C_{1-8}$alkylthio; or Ar, Ar-oxy or Ar-thio unsubstituted or substituted with 1 to 4 halogen atoms or with a $CF_3$,

$C_{1-4}$alkyl or $C_{1-4}$alkoxy group and 0 to 3 halogen atoms;

each of $R^2$ and $R^3$ is independently hydrogen; halogen; cyano; unsubstituted or halogenated $C_{1-8}$alkyl; unsubstituted or halogenated $C_{1-8}$-alkoxy; unsubstituted or halogenated $C_{1-8}$alkylthio; COOR"; Ar', Ar'-oxy or Ar'-thio unsubstituted or substituted with 1 to 4 halogenatoms or with a $C_{1-4}$alkyl, $C_{1-4}$alkoxy or $CF_3$ group and 0 to 3 halogen atoms;

or either $R^1$ and $R^2$ or $R^2$ or $R^3$ can together form a bridging group of 4 carbon atoms, saturated or unsaturated, and optionally substituted with 1 to 4 halogen atoms or with a trifluoromethyl group and 0 to 3 halogen atoms;

each of R, R' and R" is hydrogen or $C_{1-8}$alkyl;

Ar and Ar' are aromatic ring systems independently selected from naphthyl, phenyl, pyridyl and thienyl; with the proviso that where A is C-$R^4$ and B is C-$R^3$, not all of $R^1$, $R^2$, $R^3$ and $R^4$ may be hydrogen.

In the practice of the present invention, Y is preferably oxygen.

A is preferably nitrogen.

Where A is nitrogen, B is preferably C-$R^3$.

Where any of the substituents $X^1$-$X^5$ and $R^1$-$R^4$ is or comprises halogen, such halogen is conveniently selected from bromo, chloro and fluoro.

Where any of $X^1$-$X^5$ is $C_{1-8}$alkyl, it is preferably of one to four carbons and is more preferably of one or two carbons.

Where any of R, R' and R" is $C_{1-8}$alkyl, it is preferably of one to four carbons and is more preferably of one or two carbons.

The terms halogenated $C_{1-8}$alkyl, halogenated $C_{1-8}$alkoxy and halogenated $C_{1-8}$alkylthio refer to $C_{1-8}$alkyl, $C_{1-8}$alkoxy and $C_{1-8}$alkylthio, respectively, substituted by one to six, preferably one to three halogens; such halogen is preferably chloro or fluoro.

An example of a preferred halogenated $C_{1-8}$alkyl group is $CF_3$.

Ar and Ar' are preferably phenyl. Where such Ar and Ar' are substituted they may bear from 1 to 4, preferably 1 or 2 substituents. thus Ar and Ar' are preferably unsubstituted or substituted with one methyl, methoxy or $CF_3$ group and zero or one halogen atoms or with one or two halogen. Particularly preferred substituted Ar and Ar' significances are halophenyl, dihalophenyl, methylphenyl and trifluoromethylphenyl.

Preferably not more than one of $R^1$, $R^2$, $R^3$ and $R^4$ is an unnsubstituted or substituted aromatic ring system.

$X^1$ is preferably H or halogen, more preferably chloro or fluoro.

$X^2$ is preferably hydrogen or halogen; such halogen is preferably fluoro.

$X^3$ is preferably hydrogen or halogen, more preferably hydrogen.

$X^4$ conveniently signifies hydrogen, halogen, $C_{1-4}$alkyl, $CF_3$ or COOR; it is preferably hydrogen, chloro, bromo, methyl or $CF_3$.

$X^5$ is preferably hydrogen, $C_{1-4}$alkyl or halogen; it is more preferably hydrogen, chloro or methyl.

$X^5$ is preferably hydrogen or halogen, more preferably H or F.

$R^1$ conveniently signifies hydrogen, halogen, $CF_3$, unsubstituted or unsubstituted aryl, $C_{1-4}$alkoxy or together with $R^2$ forms a bridging group of 4 carbon atoms. $R^1$ is preferably hydrogen, bromo, chloro, $CF_3$, or unsubstituted or substituted phenyl, more preferably hydrogen or chloro.

$R^2$ conveniently signifies hydrogen, halogen, $CF_3$, $C_{1-4}$alkyl, COOR', cyano, unsubstituted or substituted phenyl, or together with either $R^1$ or $R^3$ forms a bridging group of 4 carbon atoms. $R^2$ is preferably hydrogen, halogen, $CF_3$ or unsubstituted or substituted phenyl. $R^2$ is more preferably hydrogen, halogen, $CF_3$ or un-, mono- or disubstituted phenyl, and is particularly H, Cl or Br.

$R^3$ conveniently signifies hydrogen, halogen, $CF_3$, $C_{1-4}$alkyl, unsubstituted or substituted phenyl or together with $R^2$ forms a bridging group of 4 carbon atoms. $R^3$ is preferably hydrogen, halogen, $CF_3$, $C_{1-4}$alkyl or un-, mono- or disubstituted phenyl, particularly H, Cl, Br, $CF_3$, 4-chlorophenyl or 4-bromophenyl.

Where $R^1$ and $R^2$ together form a bridging group this is preferably of the formula CH=CH-CH=CH; such group is preferably unsubstituted or substituted by 1 or 2 halogen atoms; such halogen is preferably chloro.

Where $R^2$ and $R^3$ together form a bridging group this is preferably of the formula $(CH_2)_4$. Such group is preferably unsubstituted or substituted by 1 or 2 halogen atoms; more preferably it is unsubstituted.

Accordingly, in a preferred subgroup of compounds of formula (A) each of $X^1$, $X^2$ and $X^3$ is independently H or halogen,

$X^4$ is H, halogen, $CH_3$ or $CF_3$,

$X^5$ is H, halogen or $CH_3$, $X^5$ is H or halogen, Y is O, A is N, B is C-$R^3$, each of $R^1$ and $R^2$ is independently H, halogen or $CF_3$,

$R^3$ is H, halogen, $C_{1-4}$alkyl or $CF_3$ whereby one of $R^1$, $R^2$ and $R^3$ may also be halophenyl, dihalophenyl, methylphenyl or trifluoromethylphenyl and/or

$R^1$ and $R^2$ together may form a bridging group of the formula CH=C-CH=CH, which group is unsubstituted or mono- or dihalogenated, or

$R^2$ and $R^3$ together form a bridging group of formula $(CH_2)_4$.

The compounds of formula (A) can have one or more asymmetric centers, geometric or positional isomers. The present invention includes each of such isomers or mixtures thereof. In the examples hereinafter such isomers are obtained as mixtures unless otherwise specified.

The compounds of formula (A) are obtained by

a) reacting a compound of formula (I)

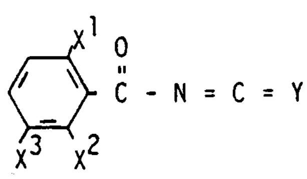

(I)

wherein $X^1$, $X^2$, $X^3$ and Y are as defined above,

with a compound of formula (II)

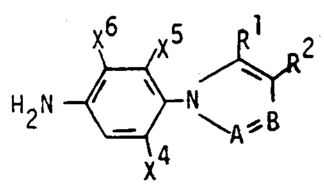

(II)

wherein $X^4$, $X^5$, $X^5$, $R^1$, $R^2$, A and B are as defined above,

or b) by reacting a benzamide of formula (III)

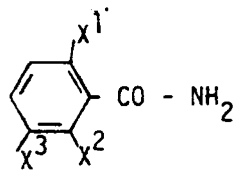 (III)

wherein $X^1$, $X^2$ and $X^3$ are as defined above,
with a compound of formula (IV)

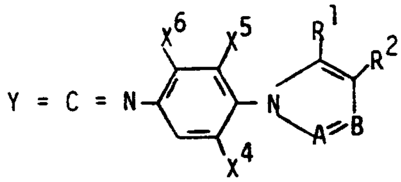 (IV)

wherein Y, $R^1$, $R^2$, $X^4$, $X^5$, $X^5$, A and B are as defined above.

The reaction of compounds of formula I with compounds of formula II (process a) may be effected under the conditions known for the preparation of N-benzoyl-N'-phenylureas from the corresponding isocyanates and anilines.

The reaction is conveniently carried out in a solvent which is inert under the reaction conditions, e.g. methylene chloride or dimethylformamide. A suitable reaction temperature may vary from -10 ° C to the boiling point of the solvent used, and preferably is about room temperature or moderately above or below room temperature, e.g. between 15 and 25 ° C.

The reaction of compounds of formula III with compounds of formula IV (process b) may be effected under the conditions known for the preparation of N-benzoyl-N'-phenylureas from the corresponding benzamides and phenylisocyanates. The reaction is conveniently carried out in a solvent which is inert under the reaction conditions. A suitable reaction temperature is from 0 ° to 120 ° C, preferably at the boiling point of the solvent used. The reaction is optionally effected in the presence of an organic base, such as pyridine.

The compounds of formula (A) may be recovered from the reaction mixture in which they are formed by working up by established procedures.

The compounds of formula I can be synthesized by treating the corresponding benzamide with oxalyl chloride or by reacting the corresponding benzoyl chloride with ammonium thiocyanate.

The aniline derivatives of formula II can be prepared by reduction of catalytic hydrogenation of the corresponding nitro compounds.

The isocyanates and isothiocyanates of formula IV can be produced by reaction of the derivatives of formula II with phosgene or thiophosgene.

The starting materials and reagents employed in the processes described herein are either known or, insofar as they are not known, may be produced in a manner analogous to the process described herein or to known processes.

The compounds of formula (A) are chitin inhibitors as indicated by tests with i.a. third instar larvae of Manduca sexta, Musca domestica, Heliothis vireseens and Spodoptera exigua, fourth instar larvae of Aedes aegypti, first instar larvae of Dermestes maculatus. They are accordingly indicated for use as pest controlling agents, particularly for the control of insects, mites and ticks.

In view of their interesting activity, particularly with regard to the level and spectrum of activity, the compounds of formula (A) offer an advantageous alternative for known chitin inhibitors, such as those disclosed in US Pat. Spec. 3 748 356 and UK Pat. Spec. 2 134 518A.

The compounds of formula (A) can be effective control agents for insects of, for example, the orders Lepidoptera, Hemiptera, Homoptera, Coleoptera, Diptera, Orthoptera and Siphonaptera, and other insects, as well as for mites and ticks of the class Acari, including mites of the families Tetranychidae and Tarsonemidae and ticks of the families Argasidae and Ixodidae. The compounds can be applied to the pest or its locus in a pest-controlling amount, usually of the order of 0.001 microgram to 100 microgram per insect, mite or tick, depending on the mode and conditions of application as well as on the pest involved.

Additionally, compounds of formula (A) may possess a repellant and/or anti-feedant action on terrestrial snails and slugs.

In the use of the compounds of formula (A) for combatting pests, a compound of formula (A), or

mixtures thereof, can conveniently be employed as pesticidal compositions in association with acceptable diluent(s) for application to the pest or its locus. Such compositions also form part of the present invention.

Suitable formulations contain from 0.01 to 99% by weight of active ingredient, from 0 to 20% of surfactant from 1 to 99.99% of diluent(s). Higher ratios of surfactant to active ingredient are sometimes desirable and are achieved by incorporation into the formulation or by tank mixing. Application forms of a composition generally contain between 0.01 and 25% by weight of active ingredient. Lower or higher levels of active ingredient can, of course, be present depending on the intended use, the physical properties of the compound and the mode of application. Concentrate forms of a composition intended to be diluted before use generally contain between 2 and 90%, preferably between 5 and 85% by weight of active ingredient.

Useful formulations of the compounds of formula (A) include dusts, granules, suspension concentrates, wettable powders, flowables and the like. They are obtained by conventional manner, e.g. by mixing a compound of formula (A) with the diluent(s) and optionally with other ingredients.

Alternatively, the compounds of formula (A) may be used in micro-encapsulated form.

The compounds of formula (A) can be combined with a cyclodextrin to make a cyclodextrin inclusion complex for application to the pest or its locus.

Agriculturally acceptable additives may be employed in the pesticidal compositions to improve the performance of the active ingredient and to reduce foaming, caking an corrosion, for example.

"Surfactant" as used herein means an agriculturally acceptable material which imparts emulsifiability, spreading, wetting, dispersibility or other surface-modifying properties. Examples of surfactants are sodium lignin sulphonate and lauryl sulfate.

"Diluent" as used herein means a liquid or solid agriculturally acceptable material used to dilute a concentrated material to a usable or desirable strength. For dusts or granules it can be e.g. talc, kaolin or diatomaceous earth, for liquid concentrate forms for example a hydrocarbon such as xylene or an alcohol such as isopropanol, and for liquid application forms i.a. water or diesel oil.

The compositions of this invention can also comprise other compounds having bilogical activity, e.g. compounds having similar or complementary pesticidal or insect growth regulating activity or compounds having antidotal, fungicidal, herbicidal or insect attractant activity.

The following examples are provided to illustrate the practice of the present invention. Temperature is given in degrees Centigrade. RT means room temperature. Parts and percentages are by weight. The symbols *, = and + when used in connection with melting points means "gas", "softens" and "decomposes" respectively. DMF means dimethyl formamide.

**COMPOSITION EXAMPLES**

| Example A: Dust | |
|---|---|
| Compound 14 | 5.1% |
| kaolin | 94.9% |

| Example B: Flowable | |
|---|---|
| Compound 14 | 48.0% |
| dispersant | 4.0% |
| thickener | 0.6% |
| antifoam | 0.1% |
| water | 41.3% |
| propylene glycol | 6.0% |

| Example C: Wettable Powder | |
|---|---|
| Compound 17 | 81.0% |
| kaolin | 14.8% |
| dispersant | 4.0% |
| wetting agent | 0.2% |

The ingredients are mixed and milled until the mean particle size is about 5 micron.

## PREPARATION OF FINAL UREAS

Example 1 : N-4-(4-chloro-1-pyrazolyl)phenyl-N'-2,6-difluorobenzoylurea

2,6-Difluorobenzoyl isocyanate (0.47 g, 2.6 mmol) is added dropwise to a solution of 4-(4-chloro-1-pyrazolyl)aniline (0.50 g, 2.6 mmol) in 8 ml of methylene chloride. The mixture is stirred for 30 min., then diluted with methylene chloride and filtered. The solid is washed with ether and dried to give N-4-(4-chloro-1-pyrazolyl)phenyl-N'-2,6-difluobenzoylurea (compound 1 under Table A).

Example 2: N-3,5-dichloro-4-(1-pyrazolyl)phenyl-N'-2,6-difluorobenzoylurea

To a solution of 3,5-dichloro-4-(1-pyrazolyl)aniline (0.17 g, 0.75 mmol) in 7 ml of methylene chloride and 1 ml of DMF is added 2,6-difluorobenzoyl isocyanate (0.14 g, 0.75 mmol). The resulting miature is stirred for 5 min., then diluted with ethyl acetate, washed with water and with brine, and dried. After the solvent is evaporated off, ether is added to the solid residue, the suspension is filtered and the solid is washed with ether and dried to give N-3,5-dichloro-4-(1-pyrazolyl)phenyl-N'-2,6-diflurobenzoylurea (compound 2 under Table A).

Example 3

Following generally the procedures of Example 1 or 2, each of the final product ureas under Tables A and B and those following Table B are prepared from the corresponding aniline and benzoyl isocyanate or benzoyl isothiocyanate intermediates.

TABLE A

Compounds of formula (A) wherein Y is O, A is N and B is $CR_3$ :

| Cpd | $X^1$ | $X^2$ | $X^3$ | $X^4$ | $X^5$ | $X^6$ | $R^1$ | $R^2$ | $R^3$ | m.p. (°C) |
|---|---|---|---|---|---|---|---|---|---|---|
| 1 | F | F | H | H | H | H | H | Cl | H | 240-242 |
| 2 | F | F | H | Cl | Cl | H | H | H | H | 211-212 |
| 3 | F | F | H | Cl | Cl | H | H | Cl | H | 229-230 |
| 4 | F | F | H | Cl | Cl | H | H | Br | H | 233-235 |
| 5 | F | F | H | Cl | Cl | H | H | $CF_3$ | $CF_3$ | 220-223 |
| 6 | F | F | H | Cl | Cl | H | H | H | Cl | 217-219 |
| 7 | F | F | H | Cl | Cl | H | H | Br | Br | 224-227 |
| 8 | F | F | H | Cl | Cl | H | H | H | 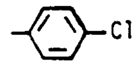 | 210-212 |
| 9 | F | F | H | Cl | Cl | H | H | 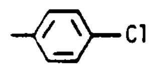 | H | 246-247 |
| 10 | F | F | H | Cl | Cl | H | H | H | $CF_3$ | 202.5-203.5 |
| 11 | F | F | H | Cl | Cl | H | H | $CF_3$ | H | 222-223 |
| 12 | F | F | H | Cl | Cl | H | Cl | H | $CF_3$ | 206-207 |
| 13 | F | F | H | Cl | Cl | H | H | H | $C(CH_3)_3$ | 204-205 |
| 14 | F | F | H | Cl | Cl | H | H | Cl | 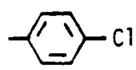 | 206.5-208.5 |
| 15 | F | F | H | Cl | Cl | H | H | Cl | Cl | 231-233 |
| 16 | F | F | H | Cl | Cl | H | H | Cl | $CF_3$ | 222.5-224 |
| 17 | F | F | H | Cl | Cl | H | Cl | Cl | $CF_3$ | 203-204 |
| 18 | F | F | H | Cl | Cl | H | $CF_3$ | H | $CF_3$ | 212.5-214 |
| 19 | F | F | H | H | H | H | $CF_3$ | H | $CF_3$ | 211.5-212 |
| 20 | F | F | H | H | H | H | Cl | H | $CF_3$ | 197-199 |
| 21 | F | F | H | H | Cl | H | H | Cl | H | 230-231 |

TABLE A (cont.)

| Cpd | $X^1$ | $X^2$ | $X^3$ | $X^4$ | $X^5$ | $X^6$ | $R^1$ | $R^2$ | $R^3$ | m.p. (°C) |
|---|---|---|---|---|---|---|---|---|---|---|
| 35 | F | F | H | Cl | H | H | H | Cl | 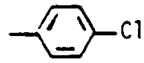 | |
| 36 | F | F | H | $CF_3$ | H | H | H | Cl | 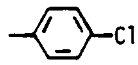 | 242-244 |
| 37 | F | F | H | $CF_3$ | H | H | Cl | H | $CF_3$ | 192.5-195 |
| 38 | F | F | H | Cl | Cl | H | Cl | Cl | Cl | 236-238 |
| 39 | F | F | H | Cl | Cl | H | H | Br | 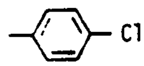 | 204-206 |
| 40 | F | F | H | Cl | Cl | H | Br | Br | Br | 249-250 |
| 41 | F | F | H | Cl | Cl | H | $CF_3$ | H | Cl | 209-211 |
| 42 | F | F | H | H | H | F | H | Cl | 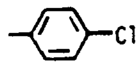 | 121.5-122.5 |
| 43 | F | F | H | Cl | H | F | H | Cl | 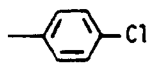 | 238-240 |
| 44 | F | F | H | $CH_3$ | H | H | H | Cl | 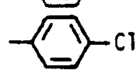 | 236-238 |
| 45 | F | F | H | Cl | H | $CH_3$ | H | Cl | 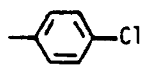 | 208-210 |
| 46 | F | F | H | Cl | Cl | H | H | 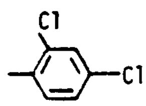 | H | 232-234 |
| 47 | F | F | H | Cl | Cl | H | H | 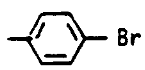 | H | 253-254 |
| 48 | F | F | H | $CH_3$ | $CH_3$ | H | H | Cl | 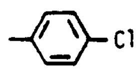 | 217-219 |
| 49 | F | F | H | Cl | Cl | H | $OCH_3$ | Cl | 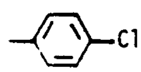 | 216-217 |
| 50 | F | F | H | Cl | Cl | H | H | $C(O)OCH_2CH_3$ | H | 213-215 |

TABLE A (cont.)

| Cpd | $X^1$ | $X^2$ | $X^3$ | $X^4$ | $X^5$ | $X^6$ | $R^1$ | $R^2$ | $R^3$ | m.p. (°C) |
|---|---|---|---|---|---|---|---|---|---|---|
| 51 | F | F | H | $C(O)OCH_3$ | H | H | H | Cl | 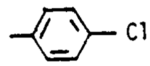 | 210-212 |
| 52 | F | F | H | Cl | H | H | Cl | H | $CF_3$ | 207.5-208.5 |
| 53 | F | F | H | Cl | H | H | H | Br | H | 226-228 |
| 54 | F | F | H | $CF_3$ | H | H | H | Br | H | 206-208 |
| 55 | F | F | H | Cl | Cl | H | H | H | 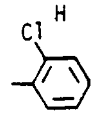 | 230-232 |
| 56 | F | F | H | Cl | Cl | H | H | H | 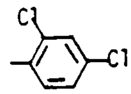 | 231-232 |
| 57 | F | F | H | Cl | Cl | H | H | H | 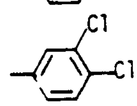 | 224-225 |
| 58 | F | F | H | Cl | Cl | H | H | H | 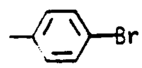 | 224-225 |
| 59 | F | F | H | Cl | Cl | H | H | H | 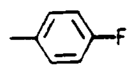 | 222-224 |
| 60 | F | F | H | Cl | Cl | H | H | H | 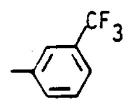 | 199-200 |
| 61 | F | F | H | Cl | Cl | H | H | H | 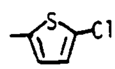 | 226-228 |
| 62 | F | F | H | Cl | Cl | H | H | H | 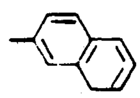 | 228-229 |
| 63 | F | F | H | Cl | Cl | H | H | CN | H | 246-248 |
| 64 | F | H | H | Cl | Cl | H | Cl | Cl | $CF_3$ | 193-194.5 |
| 65 | F | H | H | Cl | Cl | H | H | Cl | 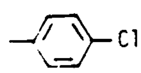 | 198-200 |

TABLE A (cont.)

| Cpd | $X^1$ | $X^2$ | $X^3$ | $X^4$ | $X^5$ | $X^6$ | $R^1$ | $R^2$ | $R^2$ | m.p. (°C) |
|---|---|---|---|---|---|---|---|---|---|---|
| 66 | F | H | H | Cl | H | H | Cl | H | $CF_3$ | 202-203 |
| 67 | F | H | H | Cl | H | H | H | Br | H | 213-215 |
| 68 | F | H | H | $CF_3$ | H | H | H | Br | H | 170-172 |
| 69 | F | H | H | Cl | Cl | H | H | H | 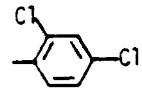 | 215-216 |
| 70 | F | H | H | Cl | Cl | H | H | H | 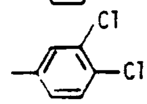 | 234-236 |
| 71 | F | H | H | Cl | Cl | H | H | H | 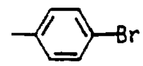 | 218-219 |
| 72 | F | H | H | Cl | Cl | H | H | H | 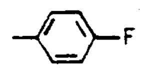 | 199-201 |
| 73 | F | H | H | Cl | Cl | H | H | H | 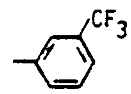 | 202-204 |
| 74 | $CH_3$ | H | H | Cl | Cl | H | Cl | Cl | $CF_3$ | 211-212.5 |
| 75 | $CH_3$ | H | H | Cl | Cl | H | H | Cl | 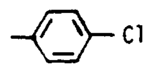 | 243-245 |
| 76 | Cl | H | F | Cl | Cl | H | Cl | Cl | $CF_3$ | 211-212.5 |
| 77 | Cl | F | H | Cl | Cl | H | Cl | Cl | $CF_3$ | 220.5-222 |
| 78 | Cl | Cl | H | Cl | Cl | H | Cl | Cl | $CF_3$ | 231.5-232.5 |
| 79 | Cl | H | H | H | H | H | H | Cl | H | |
| 80 | Cl | H | H | Cl | Cl | H | H | H | H | 200-202 |
| 81 | Cl | H | H | Cl | Cl | H | H | Cl | H | 236-238 |
| 82 | Cl | H | H | Cl | Cl | H | H | Br | H | 234-236 |
| 83 | Cl | H | H | Cl | Cl | H | H | $CF_3$ | $CF_3$ | 195-196 |
| 84 | Cl | H | H | Cl | Cl | H | H | H | Cl | 202-203 |
| 85 | Cl | H | H | Cl | Cl | H | H | Br | Br | 208-210 |

TABLE A (cont.)

| Cpd | $X^1$ | $X^2$ | $X^3$ | $X^4$ | $X^5$ | $X^6$ | $R^1$ | $R^2$ | $R^3$ | m.p. (°C) |
|---|---|---|---|---|---|---|---|---|---|---|
| 86 | Cl | H | H | Cl | Cl | H | H | H | 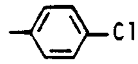 | 201.5-203 |
| 87 | Cl | H | H | Cl | Cl | H | H | 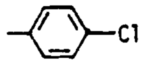 | H | 227-230 |
| 88 | Cl | H | H | Cl | Cl | H | H | H | $CF_3$ | |
| 89 | Cl | H | H | Cl | Cl | H | H | $CF_3$ | H | 207-208 |
| 90 | Cl | H | H | Cl | Cl | H | Cl | H | $CF_3$ | 209-210 |
| 91 | Cl | H | H | Cl | Cl | H | H | H | $C(CH_3)_3$ | 200-201 |
| 92 | Cl | H | H | Cl | Cl | H | H | Cl | 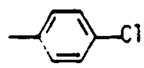 | 229-231.5 |
| 93 | Cl | H | H | Cl | Cl | H | H | Cl | Cl | 220-227 |
| 94 | Cl | H | H | Cl | Cl | H | H | Cl | $CF_3$ | 202-203 |
| 95 | Cl | H | H | Cl | Cl | H | Cl | Cl | $CF_3$ | 203.5-204.5 |
| 96 | Cl | H | H | Cl | Cl | H | $CF_3$ | H | $CF_3$ | 204-205 |
| 97 | Cl | H | H | H | H | H | $CF_3$ | H | $CF_3$ | |
| 98 | Cl | H | H | H | H | H | Cl | H | $CF_3$ | |
| 99 | Cl | H | H | H | Cl | H | H | Cl | H | |
| 100 | Cl | H | H | Cl | H | H | H | Cl | 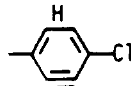 | 225-226.5 |
| 101 | Cl | H | H | $CF_3$ | H | H | H | Cl | 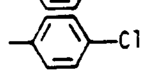 | 198-199 |
| 102 | Cl | H | H | $CF_3$ | H | H | Cl | H | $CF_3$ | 183-185 |
| 103 | Cl | H | H | Cl | Cl | H | H | Br | 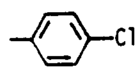 | 222-224 |
| 104 | Cl | H | H | Cl | Cl | H | Br | Br | Br | 232-233 |
| 105 | Cl | H | H | Cl | Cl | H | $CF_3$ | H | Cl | 208-209 |

TABLE A (cont.)

| Cpd | $X^1$ | $X^2$ | $X^3$ | $X^4$ | $X^5$ | $X^6$ | $R^1$ | $R^2$ | $R^3$ | m.p. (°C) |
|---|---|---|---|---|---|---|---|---|---|---|
| 106 | Cl | H | H | Cl | Cl | H | Cl | Cl | Cl | 222-224 |
| 107 | Cl | H | H | $CH_3$ | $CH_3$ | H | H | Cl | 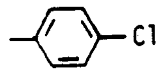 | 216-218 |
| 108 | Cl | H | H | Cl | H | H | H | Br | H | 243-245 |
| 109 | Cl | H | H | $CF_3$ | H | H | H | Br | H | 184-186 |
| 110 | Cl | H | H | Cl | Cl | H | H | H | 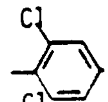 | 184-187 |
| 111 | Cl | H | H | Cl | Cl | H | H | H | 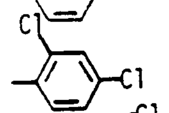 | 211-213 |
| 112 | Cl | H | H | Cl | Cl | H | H | H | 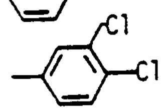 | 220-221 |
| 113 | Cl | H | H | Cl | Cl | H | H | H | 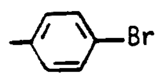 | 210-211 |
| 114 | Cl | H | H | $CH_3$ | Cl | H | H | Cl | 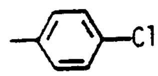 | 240-242 |
| 115 | Cl | H | H | Cl | Cl | H | H | H | 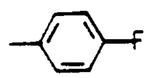 | 191-192 |
| 116 | Cl | H | H | Cl | Cl | H | H | H | 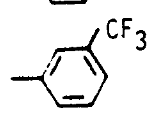 | 150-151 |
| 117 | Cl | H | H | Cl | Cl | H | H | H | 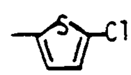 | 220-222 |
| 118 | Cl | H | H | Cl | Cl | H | H | H | 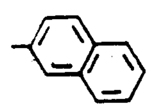 | 228-229 |
| 119 | Cl | H | H | H | Cl | H | Cl | H | $CF_3$ | 209-210 |

TABLE A (cont.)

| Cpd | $X^1$ | $X^2$ | $X^3$ | $X^4$ | $X^5$ | $X^6$ | $R^1$ | $R^2$ | $R^3$ | m.p. (°C) |
|---|---|---|---|---|---|---|---|---|---|---|
| 149 | Cl | H | H | Cl | Cl | H | 4-Br-phenyl | H | H | 218-220 |
| 150 | Cl | H | H | Cl | Cl | H | " " | Cl | H | 225-229 |
| 151 | F | F | H | Cl | Cl | H | " " | H | H | 237-238 |
| 152 | F | F | H | Cl | Cl | H | " " | Cl | H | 301-305 |
| 153 | F | F | H | Cl | Cl | H | H | H | 4-$CH_3$-$C_6H_4$ | 198-202 |
| 154 | Cl | H | H | Cl | Cl | H | H | H | 4-$CH_3$-$C_6H_4$ | 205-210 |
| 155 | H | H | H | Cl | Cl | H | H | H | 4-Br-$C_6H_4$ | 256-259 |

TABLE B

Compounds of formula (A) wherein $X^3$ and $X^6$ are H, Y is O and $R^1 + R^2$ is $CH=CW^1-CW^2=CW^3$ (with $W^3$ in ortho-position of B)

| Cpd | $X^1$ | $X^2$ | $X^4$ | $X^5$ | A | B | $W^1$ | $W^2$ | $W^3$ | m.p. (°C) |
|---|---|---|---|---|---|---|---|---|---|---|
| 23 | F | F | Cl | Cl | N | CH | H | H | H | |
| 24 | F | F | Cl | Cl | N | C-Cl | H | H | H | 235-237 |
| 25 | F | F | Cl | Cl | N | C-Cl | H | H | Cl | |
| 26 | F | F | Cl | Cl | CH | N | Cl | Cl | H | 225-228 |
| 27 | F | F | Cl | H | CH | N | Cl | Cl | H | |
| 28 | F | F | Cl | Cl | N | N | H | Cl | H | (*) |
| 29 | F | F | Cl | Cl | N | N | Cl | Cl | H | 242-244 |
| 120 | Cl | H | Cl | Cl | N | CH | H | H | H | |
| 121 | Cl | H | Cl | Cl | N | C-Cl | H | H | H | |
| 122 | Cl | H | Cl | Cl | N | C-Cl | H | H | Cl | |
| 123 | Cl | H | Cl | Cl | CH | N | Cl | Cl | H | 246-249 |
| 124 | Cl | H | Cl | H | CH | N | Cl | Cl | H | 225-228 |
| 125 | Cl | H | Cl | Cl | N | N | H | Cl | H | 103-106 |
| 126 | Cl | H | Cl | Cl | N | N | Cl | Cl | H | 222-225 |
| 127 | F | F | H | Cl | C-$CF_3$ | N | Cl | Cl | H | 218-223 |

(*) isomer A : m.p. 196-198°
isomer B : m.p. 177-181°

128. N-3,5-dichloro-4-(2-indazolyl)phenyl-N'-2,6-difluorobenzoyl urea, m.p. 209-210°;

129. N-3,5-dichloro-4-(2-indazolyl)phenyl-N'-2-chlorobenzoyl urea, m.p. 216-217°;

130. N-3,5-dichloro-4-(4,5,6,7-tetrahydroisoindol-2-yl)phenyl-N'-2,6-difluorobenzoylurea, m.p. 240-242°;

131. N-3,5-dichloro-4-(2,5-dichloro-1-pyrrolyl)phenyl-N'-2,6-difluorobenzoylurea, m.p. 206-208°;

132. N-3,5-dichloro-4-(2,3,4,5-tetrachloro-1-pyrrolyl)phenyl-N'-2,6-difluorobenzoylurea, m.p. 244-245°;

133. N-3,5-dichloro-4-(3,4-dichloro-1-pyrrolyl)phenyl-N'-2,6-difluorobenzoylurea, m.p. 236-239°;

134. N-3,5-dichloro-4-[3-chloro-4-(2,4-dichlorophenyl)-1-pyrrolyl]phenyl-N'-2,6-difluorobenzoylurea, m.p. 200-206°;

135. N-3,5-dichloro-4-(4,5,6,7-tetrahydroisoindol-2-yl)phenyl-N'-2-chlorobenzoylurea, m.p. 230-231°;

136. N-3,5-dichloro-4-(2,5-dichloro-1-pyrrolyl)phenyl-N'-2-chlorobenzoylurea;

137. N-3,5-dichloro-4-(2,3,4,5-tetrachloro-1-pyrrolyl)phenyl-N'-2-chlorobenzoylurea, m.p. 234-235°;

138. N-3,5-dichloro-4-(3,4-dichloro-1-pyrrolyl)phenyl-N'-2-chlorobenzoylurea, m.p. 220-221°;

139. N-3,5-dichloro-4-[3-chloro-4-(2,4-dichlorophenyl)-1-pyrrolyl]phenyl-N'-2-chlorobenzoylurea, m.p. 177-181°;

140. N-3,5-dichloro-4-(4,5,6,6-tetrahydroisoindol-2-yl)phenyl-N'-2-chloro-5-fluorobenzoylurea;

141. N-3,5-dichloro-4-[3,4-bis(trifluoromethyl)-1-pyrazolyl]phenyl-N'-2-chloro-5-fluorobenzoylurea;

142. N-3,5-dichloro-4-(4,5-dichloro-1-benzotriazolyl)phenyl-N'-2-chloro-5-fluorobenzoylurea;

143. N-3,5-dichloro-4-(4,5,6,7-tetrahydroisoindol-2-yl)phenyl-N'-2-chlorobenzoylthiourea;

144. N-3,5-dichloro-4-(3,4-dichloro-1-pyrrolyl)phenyl-N'-2-chlorobenzoylthiourea;

145. N-3,5-dichloro-4-[3,4-bis(trifluoromethyl)-1-pyrazolyl]phenyl-N'-2-chlorobenzoylthiourea;

146. N-3,5-dichloro-4-[4-chloro-3-(4-chlorophenyl)-1-pyrazolyl]phenyl-N'-2-chlorobenzoylthiourea;

147. N-3,5-dichloro-4-(4,5-dichloro-1-benzotriazolyl)phenyl-N'-2-chlorobenzoylthiourea;

148. N-3,5-dichloro-4-[4-chloro-3-(4-chlorophenyl)-1-pyrazolyl]phenyl-N'-2,6-difluorobenzoylurea, m.p. 166-167.5°.

## BIOLOGICAL ACTIVITY

Example 4

Early (0-24 hr) third instar larvae of the tobacco budworm, Heliothis virescens, are topically treated on the dorsal abdomen with 1 microlitre of acetone dilution of the test compound at the concentration to be tested. The treated larvae are placed on artificial diet in individual cells of a plastic grid contained in a covered plastic petri dish. The containers are held at 27°C, 16 hour photoperiod until all larvae are either dead or have molted to fifth instar larvae. In general, insecticidal activity is observed after application of from about 0.004 to 0.070 microgram test compound (4 to 70 ppm) per insect.

## Claims

**Claims for the following Contracting States : BE, CH, DE, FR, GB, IT, LI, NL**

**1.** Compounds of formula (A)

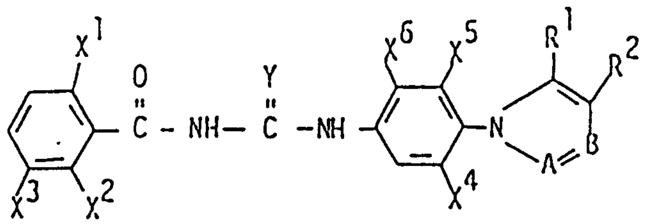

(A)

wherein, each of $X^1$, $X^2$, $X^3$ and $X^5$ is independently hydrogen, halogen or $C_{1-4}$alkyl;

$X^4$ is hydrogen, halogen, unsubstituted or halogenated $C_{1-4}$alkyl or COOR;

$X^5$ is hydrogen, halogen, $C_{1-8}$alkyl or COOR';

Y is oxygen or sulfur;

A is nitrogen or C-$R^4$;

B is nitrogen or C-$R^3$;

each of $R^1$ and $R^4$ is independently hydrogen, halogen, halogenated $C_{1-8}$-alkyl, unsubstituted or halogenated $C_{1-8}$alkoxy, unsubstituted or halogenated $C_{1-8}$alkylthio;or Ar, Ar-oxy or Ar-thio unsubstituted or substituted with 1 to 4 halogen atoms or with a $CF_3$,

$C_{1-4}$alkyl or $C_{1-4}$alkoxy group and 0 to 3 halogen atoms;

each of $R^2$ and $R^3$ is independently hydrogen; halogen; cyano; unsubstituted or halogenated $C_{1-8}$alkyl; unsubstituted or halogenated $C_{1-8}$-alkoxy; unsubstituted or halogenated $C_{1-8}$alkylthio; COOR''; Ar', Ar'-oxy or Ar'-thio unsubstituted or substituted with 1 to 4 halogenatoms or with a $C_{1-4}$alkyl, $C_{1-4}$alkoxy or $CF_3$ group and 0 to 3 halogen atoms;

or either R and $R^2$ or $R^2$ or $R^3$ can together form a bridging group of 4 carbon atoms, saturated or unsaturated, and optionally substituted with 1 to 4 halogen atoms or with a trifluoromethyl group and 0 to 3 halogen atoms;

each of R, R' and R'' is hydrogen or $C_{1-8}$alkyl;

Ar and Ar' are aromatic ring systems independently selected from naphthyl, phenyl, pyridyl and thienyl;

with the proviso that where A is C-$R^4$ and B is C-$R^3$, not all of $R^1$, $R^2$, $R^3$ and $R^4$ may be hydrogen.

**2.** A compound according to Claim 1 wherein A is nitrogen and B is C-$R^3$ where $R^3$ is as defined in Claim 1.

**3.** A compound according to Claims 1 or 2 wherein Ar and Ar' are independently unsubstituted or mono- or disubstituted with one methyl, methoxy or $CF_3$ group and zero or one halogen or with one or two halogen.

**4.** A compound according to Claim 3, wherein Y is O.

**5.** A compound according to Claim 3 or 4, wherein
each of $X^1$, $X^2$ and $X^3$ is independently H or halogen,
$X^4$ is H, halogen, $CH_3$ or $CF_3$,
$X^5$ is H, halogen or $CH_3$,
$X^5$ is H or halogen,
each of $R^1$ and $R^2$ is independently H, halogen or $CF_3$
and $R^3$ is H, halogen, $C_{1-4}$alkyl or $CF_3$ whereby one of $R^1$, $R^2$ and $R^3$ may also be halophenyl, dihalophenyl, methylphenyl or trifluoromethylphenyl and/or
$R^1$ and $R^2$ together may form a bridging group of the formula -CH=CH-CH=CH-, which group is unsubstituted or mono- or dihalogenated, or
$R^2$ and $R^3$ together form a bridging group of formula $-(CH_2)_4-$.

**6.** A compound according to Claim 5, wherein $X^1$ is halogen.

**7.** A compound according to Claim 6,
wherein $X^3$ and $X^5$ are H,
$X^4$ is halogen, $CH_3$ or $CF_3$,
$X^5$ is halogen or $CH_3$
and $R^3$ is H, halogen, $CF_3$ or halophenyl.

**8.** A compound according to Claim 7 selected from
a) N-3-5-dichloro-4-[4-chloro-3-(4-chlorophenyl)-1-pyrazolyl]-phenyl-N'-2,6-difluorobenzoylurea;
b) N-3,5-dimethyl-4-[4-chloro-3-(4-chlorophenyl)-1-pyrazoly]-phenyl-N'-2,6-difluorobenzoylurea,
c) N-3,5-dichloro-4-(4-bromo-1-pyrazolyl)-phenyl-N'-2-chlorobenzoylurea,
d) N-3,5-dichloro-4-(3,4-dibromo-1-pyrazolyl)phenyl-N'-2-chlorobenzoylurea,
e) N-3,5-dichloro-4-(4,5-dichloro-3-trifluoromethyl-1-pyrazolyl)phenyl-N'-2-chlorobenzoylurea,
f) N-3,5-dichloro-4-[3-(4-chlorophenyl)-]-1-pyrazolyl]-phenyl-N'-2-chlorobenzoylurea,
g) N-3,5-dichloro-4-(3,4,5-trichloro-1-pyrazolyl)phenyl-N'-2-chlorobenzoylurea,
h) N-3,5-dichloro-4-(4,5-dichloro-3-trifluoromethyl-1-pyrazolyl)phenyl-N'-2-fluorobenzoylurea, and
i) N-3,5-dichloro-4-[3-(4-bromophenyl)-1-pyrazolyl]-phenyl-N'-2-fluorobenzoylurea.

**9.** A compound according to claim 1 wherein $X^1$ is chloro or fluoro; each of $X^2$ and $X^3$ is independently hydrogen or fluoro; $X^4$ is hydrogen, chloro, bromo or trifluoromethyl; $X^5$ is hydrogen or chloro; $X^5$ is hydrogen or fluoro; A is nitrogen; B is C-$R^3$; and each of $R^1$, $R^2$ and $R^3$ is independently hydrogen, bromo, chloro or trifluoromethyl.

**10.** A process for preparing a compound of any one of Claims 1 to 9 which comprises
a) reacting a compound of formula (I)

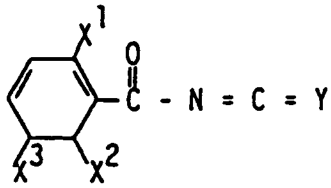

(I)

wherein $X^1$, $X^2$, $X^3$ and Y are as defined in Claim 1, with a compound of formula (II)

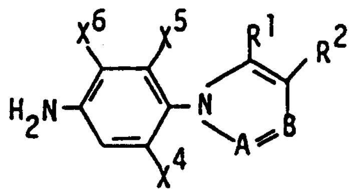

(II)

wherein $X^4$, $X^5$, $X^5$, $R^1$, $R^2$, A and B are as defined in Claim 1, or b) reacting a benzamide of formula (III)

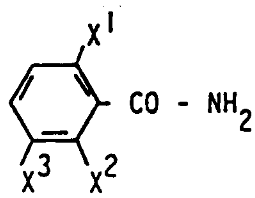

(III)

wherein $X^1$, $X^2$ and $X^3$ are as defined in Claim 1, with a compound of formula (IV)

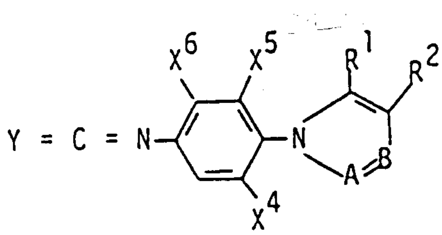

(IV)

wherein Y, $R^1$, $R^2$, $X^4$, $X^5$, $X^5$, A and B are as defined in Claim 1.

**11.** A process according to Claim 10, substantially as described herein by way of example.

**12.** A compound of formula (A) as defined in any one of Claims 1 to 9 whenever obtained by a process according to Claim 10 or 11.

**13.** A pesticidal composition comprising a compound of formula (A) as defined in any one of Claims 1 to 9 or 12 and an agriculturally acceptable diluent.

**14.** A method of combatting pests which comprises applying to the pest or its locus a pest-controlling amount of a compound of formula (A) as defined in any one of Claims 1 to 9 or 12.

**Claims for the following Contracting States : AT, ES, GR**

**1.** A pesticidal composition comprising a compound of formula (A)

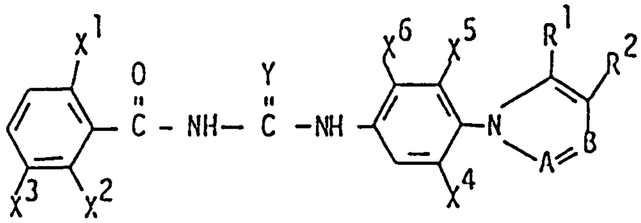

(A)

wherein, each of $X^1$, $X^2$, $X^3$ and $X^5$ is independently hydrogen, halogen or $C_{1-4}$alkyl;

$X^4$ is hydrogen, halogen, unsubstituted or halogenated $C_{1-4}$alkyl or COOR;

$X^5$ is hydrogen, halogen, $C_{1-8}$alkyl or COOR';

Y is oxygen or sulfur;

A is nitrogen or C-$R^4$;

B is nitrogen or C-$R^3$;

each of $R^1$ and $R^4$ is independently hydrogen, halogen, halogenated $C_{1-8}$-alkyl, unsubstituted or halogenated $C_{1-8}$alkoxy, unsubstituted or halogenated $C_{1-8}$alkylthio;or Ar, Ar-oxy or Ar-thio unsubstituted or substituted with 1 to 4 halogen atoms or with a $CF_3$,

$C_{1-4}$ alkyl or $C_{1-4}$alkoxy group and 0 to 3 halogen atoms;

each of $R^2$ and $R^3$ is independently hydrogen; halogen; cyano; unsubstituted or halogenated $C_{1-8}$alkyl; unsubstituted or halogenated $C_{1-8}$-alkoxy; unsubstituted or halogenated $C_{1-8}$alkylthio; COOR''; Ar', Ar'-oxy or Ar'-thio unsubstituted or substituted with 1 to 4 halogenatoms or with a $C_{1-4}$alkyl, $C_{1-4}$alkoxy or $CF_3$ group and 0 to 3 halogen atoms;

or either $R^1$ and $R^2$ or $R^2$ or $R^3$ can together form a bridging group of 4 carbon atoms, saturated or unsaturated, and optionally substituted with 1 to 4 halogen atoms or with a trifluoromethyl group and 0 to 3 halogen atoms;

each of R, R' and R'' is hydrogen or $C_{1-8}$alkyl;

Ar and Ar' are aromatic ring systems independently selected from naphthyl, phenyl, pyridyl and thienyl;

with the proviso that where A is C-$R^4$ and B is C-$R^3$, not all of $R^1$, $R^2$, $R^3$ and $R^4$ may be hydrogen, and an agriculturally acceptable diluent.

2. A composition according to Claim 1 wherein A is nitrogen and B is C-$R^3$ where $R^3$ is as defined in Claim 1.

3. A composition according to Claims 1 or 2 wherein Ar and Ar' are independently unsubstituted or mono- or disubstituted with one methyl, methoxy or $CF_3$ group and zero or one halogen or with one or two halogen.

4. A composition according to Claim 3, wherein Y is 0.

5. A composition according to Claim 3 or 4, wherein
each of $X^1$, $X^2$ and $X^3$ is independently H or halogen,

$X^4$ is H, halogen, $CH_3$ or $CF_3$,

$X^5$ is H, halogen or $CH_3$,

$X^5$ is H or halogen,

each of $R^1$ and $R^2$ is independently H, halogen or $CF_3$,

$R^3$ is H, halogen, $C_{1-4}$alkyl or $CF_3$ whereby one of $R^1$, $R^2$ and $R^3$ may also be halophenyl, dihalophenyl, methylphenyl or trifluoromethylphenyl and/or

$R^1$ and $R^2$ together may form a bridging group of the formula CH=C-CH=CH, which group is unsubstituted or mono- or dihalogenated, or

$R^2$ and $R^3$ together form a bridging group of formula $(CH_2)_4$.

6. A composition according to Claim 5, wherein $X^1$ is halogen.

7. A composition according to Claim 6,
wherein $X^3$ and $X^5$ are H,

$X^4$ is halogen, $CH_3$ or $CF_3$,

$X^5$ is halogen or $CH_3$
and $R^3$ is H, halogen, $CF_3$ or halophenyl.

**8.** A composition according to claim 1 wherein $X^1$ is chloro or fluoro; each of $X^2$ and $X^3$ is independently hydrogen or fluroro; $X^4$ is hydrogen, chloro, bromo or triflurormethyl; $X^5$ is hydrogen or chloro; $X^5$ is hydrogen or fluoro; A is nitrogen; B is C-$R^3$; and each of $R^1$, $R^2$ ad $R^3$ is independently hydrogen, bromo, chloro or trifluoromethyl.

**9.** A process for preparing a compound of Claims 1 to 7 which comprises
a) reacting a compound of formula (I)

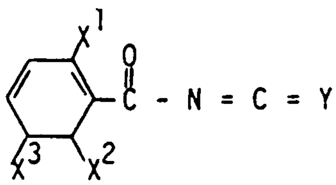

(I)

wherein $X^1$, $X^2$, $X^3$ and Y are as defined in Claim 1,
with a compound of formula (II)

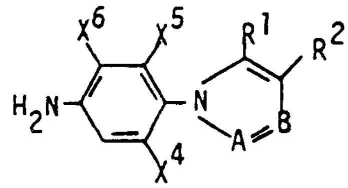

(II)

wherein $X^4$, $X^5$, $X^5$, $R^1$, $R^2$, A and B are as defined in Claim 1,
or by b) by reacting a benzamide of formula (III)

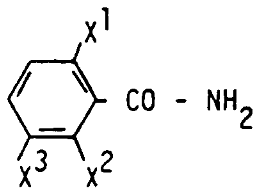

(III)

wherein $X^1$, $X^2$ and $X^3$ are as defined in Claim 1,
with a compound of formula (IV)

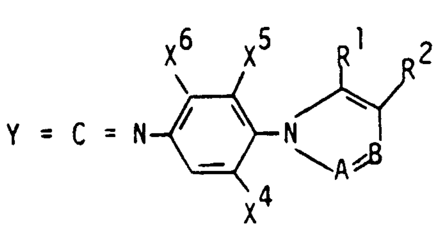

(IV)

wherein Y, $R^1$, $R^2$, $X^4$, $X^5$, $X^5$, A and B are as defined in Claim 1.

## Patentansprüche

**Patentansprüche für folgende Vertragsstaaten : BE, CH, DE, FR, GB, IT, LI, NL**

**1.** Verbindungen der Formel (A)

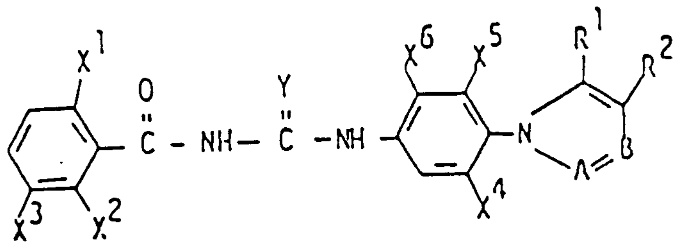

(A)

worin

$X^1$, $X^2$, $X^3$ und $X^5$ unabhängig voneinander Wasserstoff, Halogen oder $C_1$-$C_4$-Alkyl bedeuten,

$X^4$ Wasserstoff, Halogen, unsubstituiertes oder halogeniertes $C_1$-$C_4$-Alkyl oder COOR ist,

$X^5$ Wasserstoff, Halogen, $C_1$-$C_8$-Alkyl oder COOR' darstellt,

Y Sauerstoff oder Schwefel ist,

A Stickstoff oder C-$R^4$ bedeutet,

B Stickstoff oder C-$R^3$ ist,

$R^1$ und $R^4$ unabhängig voneinander Wasserstoff, Halogen, halogeniertes $C_1$-$C_8$-Alkyl, unsubstituiertes oder halogeniertes $C_1$-$C_8$-Alkoxy, unsubstituiertes oder halogeniertes $C_1$-$C_8$-Alkylthio oder Ar, Aroxy oder Ar-thio in unsubstituierter oder in durch 1 bis 4 Halogentome oder durch eine $CF_3$-, $C_1$-$C_4$-Alkyl- oder $C_1$-$C_4$-Alkoxygruppe und 0 bis 3 Halogenatome substituierter Form bedeuten,

$R^2$ und $R^3$ unabhängig voneinander Wasserstoff, Halogen, Cyano, unsubstituiertes oder halogeniertes $C_1$-$C_8$-Alkyl,unsubstituiertes oder halogeniertes $C_1$-$C_8$-Alkoxy, unsubstituiertes oder halogeniertes $C_1$-$C_8$-Alkylthio, COOR'', Ar', Ar'-oxy oder Ar'-thio in unsubstituierter oder in durch 1 bis 4 Halogenatome oder durch eine $CF_3$-, $C_1$-$C_4$-Alkyl oder $C_1$-$C_4$-Alkoxygruppe und 0 bis 3 Halogenatome substituierter Form sind,

oder entweder $R^1$ und $R^2$ oder $R^2$ und $R^3$ zusammen eine gesättigte oder ungesättigte Brücke mit 4 Kohlenstoffatomen bilden, die gegebenenfalls durch 1 bis 4 Halogenatome oder durch eine Trifluormethylgruppe und 0 bis 3 Halogenatome substituiert ist,

R, R' und R'' Wasserstoff oder $C_1$-$C_8$-Alkyl sind,

Ar und Ar' aromatische Ringsysteme bedeuten, die unabhängig aus Naphthyl, Phenyl, Pyridyl und Thienyl ausgewählt sind,

mit der Maßgabe, daß nicht alle Reste $R^1$, $R^2$, $R^3$ und $R^4$ Wasserstoff sein können, falls A für C-$R^4$ and B für C-$R^3$ stehen.

**2.** Verbindung nach Anspruch 1, worin A Stickstoff ist und B für C-$R^3$ steht, wobei $R^3$ wie im Anspruch 1 definiert ist.

**3.** Verbindung nach Anspruch 1 oder 2, worin Ar und Ar' unabhängig voneinander unsubstituiert oder durch eine $CF_3$-, Methyl- oder Methoxygruppe und 0 oder 1 Halogenatom oder durch 1 oder 2 Halogenatome monosubstituiert oder disubstituiert sind.

**4.** Verbindung nach Anspruch 3, worin Y für O steht.

**5.** Verbindung nach Anspruch 3 oder 4, worin

$X^1$, $X^2$ und $X^3$ jeweils unabhängig H oder Halogen sind,

$X^4$ für H, Halogen, $CH_3$ oder $CF_3$ steht,

$X^5$ für H, Halogen oder $CH_3$ steht,

$X^5$ für H oder Halogen steht,

$R^1$ und $R^2$ jeweils unabhängig H, Halogen oder $CF_3$ sind, und

$R^3$ für H, Halogen, $C_1$-$C_4$-Alkyl oder $CF_3$ steht,

wobei einer der Substituenten $R^1$, $R^2$ und $R^3$ auch Halogenphenyl, Dihalogenphenyl, Methylphenyl oder Trifluormethylphenyl sein kann, und/oder

wobei $R^1$ und $R^2$ zusammen eine Brücke der Formel $CH=CH-CH=CH$ bilden können, die unsubstituiert oder monohalogeniert oder dihalogeniert ist, oder

wobei $R^2$ und $R^3$ zusammen eine Brücke der Formel $(CH_2)_4$ bilden.

**6.** Verbindung nach Anspruch 5, worin $X^1$ Halogen ist.

**7.** Verbindung nach Anspruch 6, worin
$X^3$ und $X^6$ für H stehen,
$X^4$ für Halogen, $CH_3$ oder $CF_3$ steht,
$X^6$ Halogen oder $CH_3$ ist und
$R^3$ für H, Halogen, $CF_3$ oder Halogenphenyl steht.

**8.** Verbindung nach Anspruch 7, ausgewählt aus
a) N-3,5-Dichlor-4-[4-chlor-3-(4-chlorphenyl)-1-pyrazolyl]-phenyl-N'-2,6-difluorbenzoylharnstoff,
b) N-3,5-Dimethyl-4-[4-chlor-3-(4-chlorphenyl)-1-pyrazolyl]-phenyl-N'-2,6-difluorbenzoylharnstoff,
c) N-3,5-Dichlor-4-(4-brom-1-pyrazolyl)-phenyl-N'-2-chlorbenzoylharnstoff,
d) N-3,5-Dichlor-4-(3,4-dibrom-1-pyrazolyl)-phenyl-N'-2-chlorbenzoylharnstoff,
e) N-3,5-Dichlor-4-(4,5-dichlor-3-trifluormethyl-1-pyrazolyl)-phenyl-N'-2-chlorbenzoylharnstoff,
f) N-3,5-Dichlor-4-[3-(4-chlorphenyl)-1-pyrazolyl]-phenyl-N'-2-chlorbenzoylharnstoff,
g) N-3,5-Dichlor-4-(3,4,5-trichlor-1-pyrazolyl)-phenyl-N'-2-chlorbenzoylharnstoff,
h) N-3,5-Dichlor-4-(4,5-dichlor-3-trifluormethyl-1-pyrazolyl)-phenyl-N'-2-fluorbenzoylharnstoff und
i) N-3,5-Dichlor-4-[3-(4-bromphenyl)-1-pyrazolyl]-phenyl-N'-2-fluorbenzoylharnstoff.

**9.** Verbindung nach Anspruch 1, worin $X^1$ Chlor oder Fluor ist, $X^2$ und $X^3$ jeweils unabhängig Wasserstoff oder Fluor bedeutet, $X^4$ Wasserstoff, Chlor, Brom oder Trifluormethyl ist, $X^6$ Wasserstoff oder Chlor darstellt, $X^6$ Wasserstoff oder Fluor ist, A Stickstoff bedeutet, B für C-$R^3$ steht und $R^1$, $R^2$ sowie $R^3$ jeweils unabhängig Wasserstoff, Brom, Chlor oder Trifluormethyl sind.

**10.** Verfahren zur Herstellung einer Verbindung nach einem der Ansprüche 1 bis 9, gekennzeichnet durch
a) Umsetzung einer Verbindung der Formel (I)

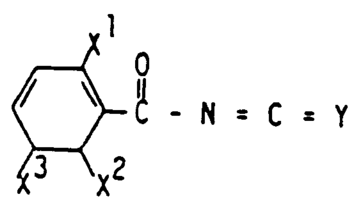
(I)

worin $X^1$, $X^2$, $X^3$ und Y wie im Anspruch 1 definiert sind,
mit einer Verbindung der Formel (II)

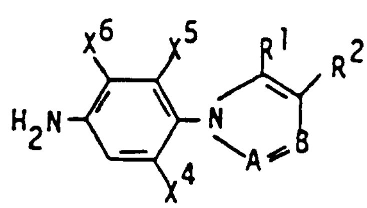
(II)

worin $X^4$, $X^6$, $X^6$, $R^1$, $R^2$, A und B wie im Anspruch 1 definiert sind, oder
b) Umsetzung eines Benzamids der Formel (III)

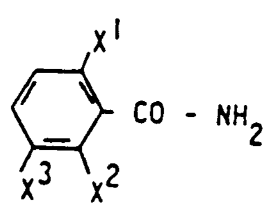
(III)

worin $X^1$, $X^2$ und $X^3$ wie im Anspruch 1 definiert sind,

mit einer Verbindung der Formel (IV)

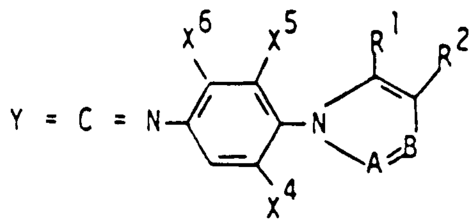

(IV)

worin Y, $R^1$, $R^2$, $X^4$, $X^5$, $X^5$, A und B wie im Anspruch 1 definiert sind.

**11.** Verfahren nach Anspruch 10, wie im wesentlichen hierin durch ein Beispiel beschrieben.

**12.** Verbindung der Formel (A) gemäß Definition nach einem der Ansprüche 1 bis 9, erhalten durch ein Verfahren gemäß Anspruch 10 oder 11.

**13.** Schädlingsbekämpfungsmittel aus einer Verbindung der Formel (A) gemäß Definition nach irgendeinem der Ansprüche 1 bis 9 oder 12 und einem landwirtschaftlich unbedenklichen Verdünnungsmittel.

**14.** Verfahren zur Bekämpfung von Schädlingen, dadurch gekennzeichnet, daß man die Schädlinge oder ihren Lebensraum mit einer schädlingsbekämpfenden Menge einer Verbindung der Formel (A) gemäß Definition nach irgendeinem der Ansprüche 1 bis 9 oder 12 behandelt.

**Patentansprüche für folgende Vertragsstaaten : AT, ES, GR**

**1.** Schädlingsbekämpfungsmittel, dadurch gekennzeichnet, daß es eine Verbindung der Formel (A)

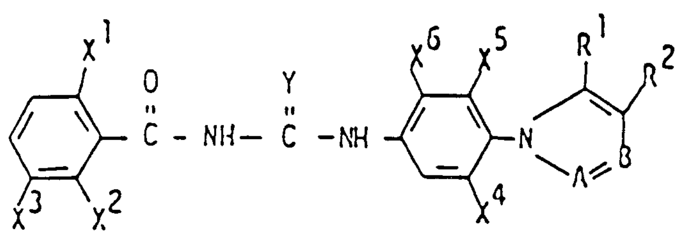

(A)

worin

$X^1$, $X^2$, $X^3$ und $X^5$ unabhängig voneinander Wasserstoff, Halogen oder $C_1$-$C_4$-Alkyl bedeuten,

$X^4$ Wasserstoff, Halogen, unsubstituiertes oder halogeniertes $C_1$-$C_4$-Alkyl oder COOR ist,

$X^5$ Wasserstoff, Halogen, $C_1$-$C_8$-Alkyl oder COOR' darstellt,

Y Sauerstoff oder Schwefel ist,

A Stickstoff oder C-$R^4$ bedeutet,

B Stickstoff oder C-$R^3$ ist,

$R^1$ und $R^4$ unabhängig voneinander Wasserstoff, Halogen, halogeniertes $C_1$-$C_8$-Alkyl, unsubstituiertes oder halogeniertes $C_1$-$C_8$-Alkoxy, unsubstituiertes oder halogeniertes $C_1$-$C_8$-Alkylthio oder Ar, Aroxy oder Ar-thio in unsubstituierter oder in durch 1 bis 4 Halogentome oder durch eine $CF_3$-, $C_1$-$C_4$-Alkyl- oder $C_1$-$C_4$-Alkoxygruppe und 0 bis 3 Halogenatome substituierter Form bedeuten,

$R^2$ und $R^3$ unabhängig voneinander Wasserstoff, Halogen, Cyano, unsubstituiertes oder halogeniertes $C_1$-$C_8$-Alkyl,unsubstituiertes oder halogeniertes $C_1$-$C_8$-Alkoxy, unsubstituiertes oder halogeniertes $C_1$-$C_8$-Alkylthio, COOR'', Ar', Ar'-oxy oder Ar'-thio in unsubstituierter oder in durch 1 bis 4 Halogenatome oder durch eine $CF_3$-, $C_1$-$C_4$-Alkyl oder $C_1$-$C_4$-Alkoxygruppe und 0 bis 3 Halogenatome substituierter Form sind,

oder entweder $R^1$ und $R^2$ oder $R^2$ und $R^3$ zusammen eine gesättigte oder ungesättigte Brücke mit 4 Kohlenstoffatomen bilden, die gegebenenfalls durch 1 bis 4 Halogenatome oder durch eine Trifluormethylgruppe und 0 bis 3 Halogenatome substituiert ist,

R, R' und R'' Wasserstoff oder $C_1$-$C_8$-Alkyl sind,

Ar und Ar' aromatische Ringsysteme bedeuten, die unabhängig aus Naphthyl, Phenyl, Pyridyl und Thienyl ausgewählt sind,

mit der Maßgabe, daß nicht alle Reste $R^1$, $R^2$, $R^3$ und $R^4$ Wasserstoff sein können, falls A für C-$R^4$ und B für C-$R^3$ stehen,

und ein landwirtschaftlich unbedenkliches Verdünnungsmittel enthält.

**2.** Mittel nach Anspruch 1, worin A Stickstoff ist und B für C-$R^3$ steht, wobei $R^3$ wie im Anspruch 1 definiert ist.

**3.** Mittel nach Anspruch 1 oder 2, worin Ar und Ar' unabhängig voneinander unsubstituiert oder durch eine $CF_3$-, Methyl- oder Methoxygruppe und 0 oder 1 Halogenatom oder durch 1 oder 2 Halogenatome monosubstituiert oder disubstituiert sind.

**4.** Mittel nach Anspruch 3, worin Y für O steht.

**5.** Mittel nach Anspruch 3 oder 4, worin

$X^1$, $X^2$ und $X^3$ jeweils unabhängig H oder Halogen sind,

$X^4$ für H, Halogen, $CH_3$ oder $CF_3$ steht,

$X^5$ für H, Halogen oder $CH_3$ steht,

$X^5$ für H oder Halogen steht,

$R^1$ und $R^2$ jeweils unabhängig H, Halogen oder $CF_3$ sind, und

$R^3$ für H, Halogen, $C_1$-$C_4$-Alkyl oder $CF_3$ steht,

wobei einer der Substituenten $R^1$, $R^2$ und $R^3$ auch Halogenphenyl, Dihalogenphenyl, Methylphenyl oder Trifluormethylphenyl sein kann, und/oder

wobei $R^1$ und $R^2$ zusammen eine Brücke der Formel CH=CH-CH=CH bilden können, die unsubstituiert oder monohalogeniert oder dihalogeniert ist, oder

wobei $R^2$ und $R^3$ zusammen eine Brücke der Formel $(CH_2)_4$ bilden.

**6.** Mittel nach Anspruch 5, worin $X^1$ Halogen ist.

**7.** Mittel nach Anspruch 6, worin

$X^3$ und $X^5$ für H stehen,

$X^4$ für Halogen, $CH_3$ oder $CF_3$ steht,

$X^5$ Halogen oder $CH_3$ ist und

$R^3$ für H, Halogen, $CF_3$ oder Halogenphenyl steht.

**8.** Mittel nach Anspruch 1, worin $X^1$ Chlor oder Fluor ist, $X^2$ und $X^3$ jeweils unabhängig Wasserstoff oder Fluor bedeutet, $X^4$ Wasserstoff, Chlor, Brom oder Trifluormethyl ist, $X^5$ Wasserstoff oder Chlor darstellt, $X^5$ Wasserstoff oder Fluor ist, A Stickstoff bedeutet, B für C-$R^3$ steht und $R^1$, $R^2$ sowie $R^3$ jeweils unabhängig Wasserstoff, Brom, Chlor oder Trifluormethyl sind.

**9.** Verfahren zur Herstellung einer Verbindung nach Anspruch 1 bis 7, gekennzeichnet durch

a) Umsetzung einer Verbindung der Formel (I)

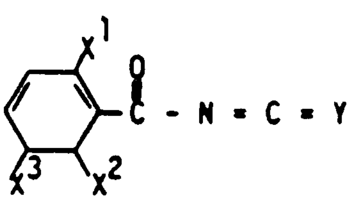

(I)

worin $X^1$, $X^2$, $X^3$ und Y wie im Anspruch 1 definiert sind,

mit einer Verbindung der Formel (II)

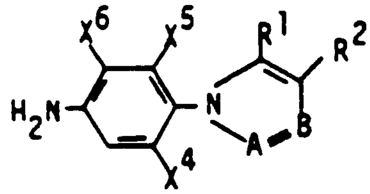

(II)

worin $X^4$, $X^5$, $X^5$, $R^1$, $R^2$, A und B wie im Anspruch 1 definiert sind, oder
b) Umsetzung eines Benzamids der Formel (III)

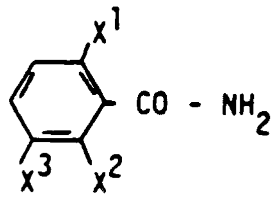

(III)

worin $X^1$, $X^2$ und $X^3$ wie im Anspruch 1 definiert sind,
mit einer Verbindung der Formel (IV)

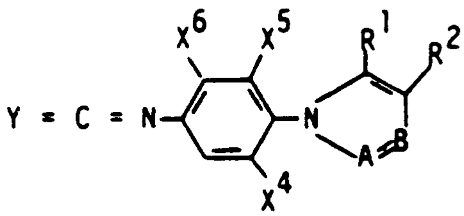

(IV)

worin Y, $R^1$, $R^2$, $X^4$, $X^5$, $X^5$, A und B wie im Anspruch 1 definiert sind.

**Revendications**

**Revendications pour les Etats contractants suivants : BE, CH, DE, FR, GB, IT, LI, NL**

**1.** Les composés de formule (A)

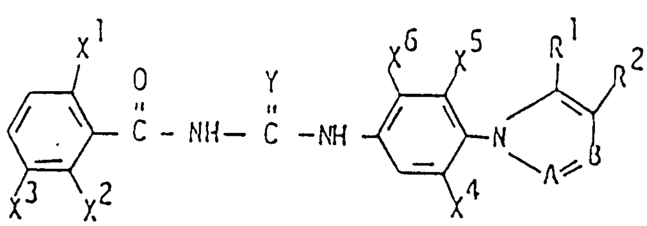

(A)

dans laquelle

| | |
|---|---|
| chaque $X^1$, $X^2$, $X^3$ et $X^5$ | signifie indépendamment l'hydrogène, un halogène ou un groupe alkyle en $C_1$-$C_4$, |
| $X^4$ | signifie l'hydrogène, un halogène, un groupe alkyle en $C_1$-$C_4$ non substitué ou halogéné ou un groupe COOR, |
| $X^5$ | signifie l'hydrogène, un halogène, un groupe alkyle en $C_1$-$C_8$ ou un groupe COOR', |
| Y | signifie l'oxygène ou le soufre, |
| A | signifie l'azote ou C-$R^4$, |
| B | signifie l'azote ou C-$R^3$, |
| chaque $R^1$ et $R^4$ | signifie indépendamment l'hydrogène, un halogène, un groupe alky- |

le en $C_1$-$C_8$ halogéné, un groupe alcoxy en $C_1$-$C_8$ non substitué ou halogéné, un groupe alkylthio en $C_1$-$C_8$ non substitué ou halogéné ou un groupe Ar, Ar-oxy ou Ar-thio non substitué ou substitué par 1 à 4 atomes d'halogène ou par un groupe $CF_3$, alkyle en $C_1$-$C_4$ ou alcoxy en $C_1$-$C_4$ et par 0 à 3 atomes d'halogène,

| | |
|---|---|
| chaque $R^2$ et $R^3$ | signifie indépendamment l'hydrogène, un halogène, un groupe cyano, un groupe alkyle en $C_1$-$C_8$ non substitué ou halogéné, un groupe alcoxy en $C_1$-$C_8$ non substitué ou halogéné, un groupe alkylthio en $C_1$-$C_8$ non substitué ou halogéné; un groupe COOR'' ou un groupe Ar', Ar'-oxy ou Ar'-thio non substitué ou substitué par 1 à 4 atomes d'halogène ou par un groupe alkyle en $C_1$-$C_4$, alcoxy en $C_1$-$C_4$ ou $CF_3$ et par 0 à 3 atomes d'halogène, |
| ou bien $R^1$ et $R^2$ ou $R^2$ et $R^3$ | peuvent former ensemble un groupe formant pont contenant 4 atomes de carbone, saturé ou insaturé et éventuellement substitué par 1 à 4 atomes d'halogène ou par un groupe trifluorométhyle et par 0 à 3 atomes d'halogène, |
| chaque R, R' et R'' | signifie l'hydrogène ou un groupe alkyle en $C_1$-$C_8$, |
| Ar et Ar' | signifient des systèmes cycliques aromatiques choisis indépendamment parmi les groupes naphtyle, phényle, pyridyle et thiényle, |

$R^1$, $R^2$, $R^3$ et $R^4$ ne pouvant tous signifier l'hydrogène lorsque A signifie C-$R^4$ et a signifie C-$R^3$.

**2.** Un composé selon la revendication 1, dans lequel A signifie l'azote et B signifie C-$R^3$ où $R^3$ est tel que défini à la revendication 1.

**3.** Un composé selon la revendication 1 ou 2, dans lequel Ar et Ar' indépendamment sont non substitués ou mono- ou disubstitués par un groupe méthyle, méthoxy ou $CF_3$ et par 0 à 1 atome d'halogène ou par 1 à 2 atomes d'halogène.

**4.** Un composé selon la revendication 3, dans lequel Y signifie 0.

**5.** Un composé selon la revendication 3 ou 4, dans lequel

| | |
|---|---|
| chaque $X^1$, $X^2$ et $X^3$ | signifie indépendamment H ou un halogène, |
| $X^4$ | signifie H, un halogène $CH_3$ ou $CF_3$, |
| $X^5$ | signifie H, un halogène ou $CH_3$, |
| $X^5$ | signifie H ou un halogène, |
| chaque $R^1$ et $R_2$ | signifie indépendamment H, un halogène ou $CF_3$, et |
| $R^3$ | signifie H, un halogène, un groupe alkyle en $C_1$-$C_4$ ou $CF_3$, l'un de $R^1$ $R^2$ et $R^3$ pouvant également signifier un groupe halophényle, dihalophényle, méthylphényle ou trifluorométhylphényle et/ou |
| $R^1$ et $R^2$ | peuvent former ensemble un groupe formant pont de formule $-CH=CH-CH=CH-$, lequel groupe est non substitué ou mono- ou dihalogéné, ou |
| $R^2$ et $R^3$ | forment ensemble un groupe formant pont de formule $-(CH_2)_4-$. |

**6.** Un composé selon la revendication 5, dans lequel $X^1$ signifie un halogène.

**7.** Un composé selon la revendication 6, dans lequel

| | |
|---|---|
| $X^3$ et $X^5$ | signifient H, |
| $X^4$ | signifie un halogène, $CH_3$ ou $CF_3$, |
| $X^5$ | signifie un halogène ou $CH_3$, et |
| $R^3$ | signifie H, un halogène, $CF_3$ ou halophényle. |

**8.** Un composé selon la revendication 7 choisi parmi

a) la N-3,5-dichloro-4-[4-chloro-3-(4-chlorophényl)-1-pyrazolyl]-phényl-N'-2,6-difluorobenzoylurée,
b) la N-3,5-diméthyl-4-[4-chloro-3-(4-chlorophényl)-1-pyrazolyl]-phényl-N'-2,6-difluorobenzoylurée,
c) la N-3,5-dichloro-4-(4-bromo-1-pyrazolyl)-phényl-N'-2-chlorobenzoylurée,
d) la N-3,5-dichloro-4-(3,4-dibromo-1-pyrazolyl)-phényl-N'-2-chlorobenzoylurée,
e) la N-3,5-dichloro-4-(4,5-dichloro-3-trifluorométhyl-1-pyrazolyl)-phényl-N'-2-chlorobenzoylurée.
f) la N-3,5-dichloro-4-[3-(4-chlorophényl)-1-pyrazolyl]-phényl-N'-2-chlorobenzoylurée,

g) la N-3,5-dichloro-4-(3,4,5-trichloro-1-pyrazolyl)-phényl-N'-2-chlorobenzoylurée,
h) la N-3,5-dichloro-4-(4,5-dichloro-3-trifluorométhyl-1-pyrazolyl)-phényl-N'-2-fluorobenzoylurée, et
i) la N-3,5-dichloro-4-[3-(4-bromophényl)-1-pyrazolyl]-phényl-N'-2-fluorobenzoylurée.

**9.** Un composé selon la revendication 1, dans lequel $X^1$ signifie chloro ou fluoro, chaque $X^2$ et $X^3$ signifie indépendamment l'hydrogène ou fluoro; $X^4$ signifie l'hydrogène, chloro, bromo ou trifluorométhyle; $X^5$ signifie l'hydrogène ou chloro; $X^5$ signifie l'hydrogène ou fluoro; A signifie l'azote; B signifie C-$R^3$; et chaque $R^1$ $R^2$ et $R^3$ signifie indépendamment l'hydrogène, bromo, chloro ou trifluorométhyle.

**10.** Un procédé de préparation d'un composé selon l'une quelconque des revendications 1 à 9, qui comprend

a) la réaction d'un composé de formule (I)

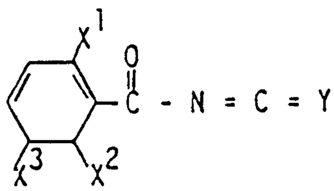

(I)

dans laquelle $X^1$, $X^2$, $X^3$ et Y sont tels que définis à la revendication 1,
avec un composé de formule (II)

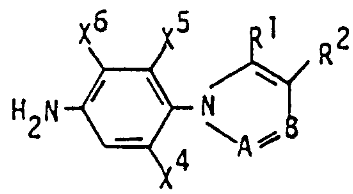

(II)

dans laquelle $X^4$, $X^5$, $X^5$, $R^1$, $R^2$, A et B sont tels que définis à la revendication 1, ou
b) la réaction d'un benzamide de formule (III)

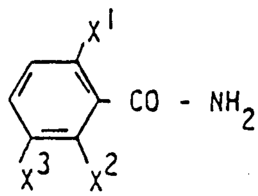

(III)

dans laquelle $X^1$, $X^2$ et $X^3$ sont tels que définis à la revendication 1,
avec un composé de formule (IV)

-22-

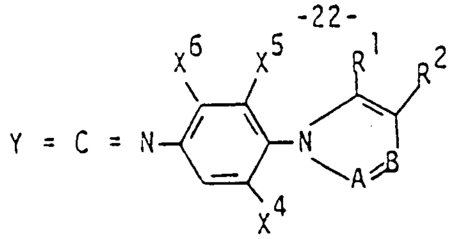

(IV)

dans laquelle Y, $R^1$, $R^2$, $X^4$, $X^5$, $X^5$, A et B sont tels que définis à la revendication 1.

**11.** Un procédé selon la revendication 10, essentiellement tel que décrit ici aux exemples.

**12.** Un composé de formule (A) tel que défini à l'une quelconque des revendications 1 à 9, lorsqu'il est obtenu par un procédé selon la revendication 10 ou 11.

**13.** Une composition pesticide comprenant un composé de formule (A) tel que défini à l'une quelconque des revendications 1 à 9 ou 12 et un diluant acceptable en agriculture.

**14.** Une méthode pour combattre les organismes nuisibles, qui comprend l'application sur l'organisme nuisible ou son habitat d'une quantité d'un composé de formule (A) tel que défini à l'une quelconque des revendications 1 à 9 ou 12, combattant l'organisme nuisible.

**Revendications pour les Etats contractants suivants : AT, ES, GR**

**1.** Une composition pesticide comprenant un composé de formule (A)

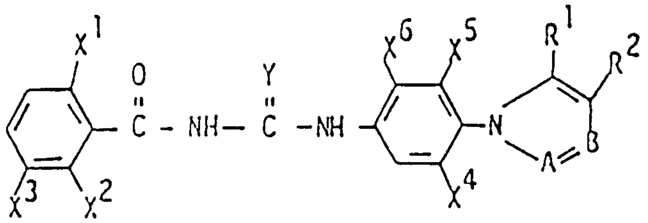

(A)

dans laquelle

| | |
|---|---|
| chaque $X^1$, $X^2$, $X^3$ et $X^5$ | signifie indépendamment l'hydrogène, un halogène ou un groupe alkyle en $C_1$-$C_4$, |
| $X^4$ | signifie l'hydrogène, un halogène, un groupe alkyle en $C_1$-$C_4$ non substitué ou halogéné ou un groupe COOR, |
| $X^5$ | signifie l'hydrogène, un halogène, un groupe alkyle en $C_1$-$C_8$ ou un groupe COOR', |
| Y | signifie l'oxygène ou le soufre, |
| A | signifie l'azote ou C-$R^4$, |
| B | signifie l'azote ou C-$R^3$, |
| chaque $R^1$ et $R^4$ | signifie indépendamment l'hydrogène, un halogène, un groupe alkyle en $C_1$-$C_8$ halogéné, un groupe alcoxy en $C_1$-$C_8$ non substitué ou halogéné, un groupe alkylthio en $C_1$-$C_8$ non substitué ou halogéné ou un groupe Ar, Ar-oxy ou Ar-thio non substitué ou substitué par 1 à 4 atomes d'halogène ou par un groupe $CF_3$ alkyle en $C_1$-$C_4$ ou alcoxy en $C_1$-$C_4$ et par 0 à 3 atomes d'halogène, |
| chaque $R^2$ et $R^3$ | signifie indépendamment l'hydrogène, un halogène, un groupe cyano, un groupe alkyle en $C_1$-$C_8$ non substitué ou halogéné, un groupe alcoxy en $C_1$-$C_8$ non substitué ou halogéné, un groupe alkylthio en $C_1$-$C_8$ non substitué ou halogéné; un groupe COOR" ou un groupe Ar', Ar'-oxy ou Ar'-thio non substitué ou substitué par 1 à 4 atomes d'halogène ou par un groupe alkyle en $C_1$-$C_4$, alcoxy en $C_1$-$C_4$ ou $CF_3$ et par 0 à 3 atomes d'halogène, |
| ou bien $R^1$ et $R^2$ ou $R^2$ et $R^3$ | peuvent former ensemble un groupe formant pont contenant 4 atomes de carbone, saturé ou insaturé et éventuellement substitué par 1 à 4 atomes d'halogène ou par un groupe trifluorométhyle et par 0 à 3 atomes d'halogène, |
| chaque R, R' et R" | signifie l'hydrogène ou un groupe alkyle en $C_1$-$C_8$, |
| Ar et Ar' | signifient des systèmes cycliques aromatiques choisis indépendamment parmi les groupes naphtyle, phényle, pyridyle et thiényle, |

$R^1$, $R^2$, $R^3$ et $R^4$ ne pouvant tous signifier l'hydrogène lorsque A signifie C-$R^4$ et B signifie C-$R^3$, et un diluant acceptable en agriculture.

**2.** Une composition selon la revendication 1, dans laquelle A signifie l'azote et B signifie C-$R^3$ où $R^3$ est tel que défini à la revendication 1.

3. Une composition selon la revendication 1 ou 2, dans laquelle Ar et Ar' indépendamment sont non substitués ou mono- ou disubstitués par un groupe méthyle, méthoxy ou $CF_3$ et par 0 ou 1 halogène ou par 1 ou 2 atomes d'halogène.

4. Une composition selon la revendication 3, dans laquelle Y signifie 0.

5. Une composition selon la revendication 3 ou 4, dans laquelle

| | |
|---|---|
| chaque $X^1$, $X^2$ et $X^3$ | signifie indépendamment H ou un halogène, |
| $X^4$ | signifie H, un halogène, $CH_3$ ou $CF_3$, |
| $X^5$ | signifie H, un halogène ou $CH_3$, |
| $X^5$ | signifie H ou un halogène, |
| chaque $R^1$ et $R_2$ | signifie indépendamment H, un halogène ou $CF_3$, |
| $R^3$ | signifie H, un halogène, un groupe alkyle en $C_1$-$C_4$ ou $CF_3$, l'un de $R^1$, $R^2$ et $R^3$ pouvant également signifier un groupe halophényle, dihalophényle, méthylphényle ou trifluorométhylphényle et/ou |
| $R^1$ et $R^2$ | peuvent former ensemble un groupe formant pont de formule -CH=CH-CH=CH-, lequel groupe est non substitué ou mono- ou dihalogéné, ou |
| $R^2$ et $R^3$ | forment ensemble un groupe formant pont de formule, $-(CH_2)_4-$. |

6. Une composition selon la revendication 5, dans laquelle $X^1$ signifie un halogène.

7. Une composition selon la revendication 6, dans laquelle

| | |
|---|---|
| $X^3$ et $X^5$ | signifient H, |
| $X^4$ | signifie un halogène, $CH_3$ ou $CF_3$, |
| $X^5$ | signifie un halogène ou $CH_3$, et |
| $R^3$ | signifie H, un halogène, $CF_3$ ou halophényle. |

8. Une composition selon la revendication 1, dans laquelle $X^1$ signifie chloro ou fluoro; chaque $X^2$ et $X^3$ signifie indépendamment l'hydrogène ou fluoro; $X^4$ signifie l'hydrogène, chloro, bromo ou trifluorométhyle; $X^5$ signifie l'hydrogène ou chloro; $X^5$ signifie l'hydrogène ou fluoro; A signifie l'azote; B signifie C-$R^3$ et chaque $R^1$, $R^2$ et $R^3$ signifie indépendamment l'hydrogène, bromo, chloro ou trifluorométhyle.

9. Un procédé de préparation d'un composé selon les revendications 1 à 7, qui comprend

a) la réaction d'un composé de formule (I)

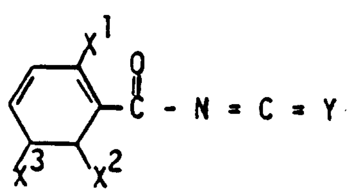

(I)

dans laquelle $X^1$, $X^2$, $X^3$ et Y sont tels que définis à la revendication 1,
avec un composé de formule (II)

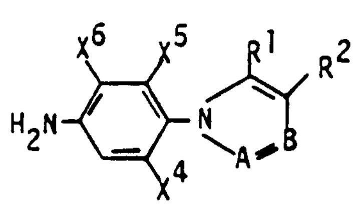

(II)

dans laquelle $X^4$, $X^5$, $X^5$, $R^1$, $R^2$, A et B sont tels que définis à la revendication 1, ou
b) la réaction d'un benzamide de formule (III)

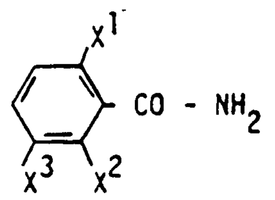

(III)

dans laquelle $X^1$, $X^2$ et $X^3$ sont tels que définis à la revendication 1, avec un composé de formule (IV)

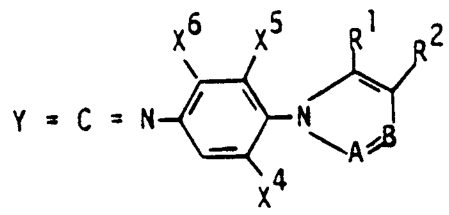

(IV)

dans laquelle Y, $R^1$, $R^2$, $X^4$, $X^5$, $X^5$, A et B sont tels que définis à la revendication 1.